# EUROPEAN PATENT SPECIFICATION

(11) **EP 4 072 798 B1**
(45) Date of publication and mention of the grant of the patent: **26.07.2023**
(21) Application number: 20816264.4
(22) Date of filing: 04.12.2020
(51) Int. Cl.: B26B 19/38, A45D 24/00, A45D 44/00

(54) **HAIR REMOVAL INSTRUCTIONS**
ANLEITUNG ZUR HAARENTFERNUNG
COMPOSITION D'ÉPILATION

(30) Priority: 11.12.2019 EP 19215228
(43) Date of publication of application: 19.10.2022
(73) Proprietor: Koninklijke Philips N.V., 5656 AG Eindhoven (NL)
(72) Inventor: PALERO, Jonathan, Alambra, 5656 AE Eindhoven (NL); VARGHESE, Babu, 5656 AE Eindhoven (NL); BOURQUIN, Yannyk, Parulian, Julian, 5656 AE Eindhoven (NL); AKKERMANS, Steffie, Petronella, 5656 AE Eindhoven (NL); BUIL, Vincentius, Paulus, 5656 AE Eindhoven (NL); DURACHER, Lucie, Tamara, Christine, 5656 AE Eindhoven (NL)
(74) Representative: Philips Intellectual Property & Standards
(86) International application number: PCT/EP2020/084757
(87) International publication number: WO 2021/115976

(56) References cited:
- WO-A1-2015/068068
- WO-A1-2017/148941
- WO-A1-2018/149738
- WO-A1-2019/011523

## Description

### TECHNICAL FIELD OF THE INVENTION

The invention relates to a method, apparatus and tangible machine-readable medium for providing hair removal instructions. An example of prior art is given by the patent documentation WO 2019/011523 A1.

### BACKGROUND OF THE INVENTION

Hair removal techniques such as shaving may cause skin irritation. However, a user may not be aware of the optimal hair removal technique for reducing skin irritation due to hair removal. A user may have difficulty establishing the optimal hair removal technique since they may find it difficult or be unable to determine whether or not their hair removal technique delivers optimal results and/or they may not be aware of any better techniques for delivering optimal results.

Accordingly, an object is to provide user guidance to improve hair removal results. Another object is to reduce skin irritation due to hair removal.

### SUMMARY OF THE INVENTION

Aspects or embodiments described herein relate to providing user guidance to improve hair removal results and/or reduce skin irritation due to hair removal. Aspects or embodiments described herein may obviate one or more problems associated with hair removal.

In a first aspect, a computer-implemented method is described. The method comprises obtaining an indication comprising a skin parameter of a user. The indication further comprises an interaction between the user's skin and a hair removal unit. The method further comprises determining a position of the hair removal unit relative to the user's skin. The method further comprises determining a hair removal instruction for the user based on the indication and the position. The method further comprises causing a user interface to provide the hair removal instruction for the user.

In some embodiments, obtaining the indication comprising the skin parameter of the user comprises accessing skin parameter data for the user determined based on imaging data of the user's skin.

In some embodiments, the method comprises causing an imaging device to acquire the imaging data prior to a user hair removal session, to determine pre-hair removal skin parameter data.

In some embodiments, the method comprises causing the imaging device to acquire the imaging data during and/or after the user hair removal session, to determine present and/or post-hair removal skin parameter data. The method may further comprise generating skin parameter data based on the imaging data. The method may further comprise determining a skin parameter map for the user based on a comparison between the pre-hair removal and present and/or post-hair removal skin parameter data.

In some embodiments, the skin parameter comprises a visible skin irritation indicator. The skin parameter may be based on whether or not the comparison identifies any change in the visible skin irritation indicator between the pre-hair removal and present and/or post-hair removal skin parameter data.

In some embodiments, the indication further comprises a hair parameter of the user. The method may further comprise determining the hair removal instruction taking into account the hair parameter.

In some embodiments, determining the hair removal instruction comprises accessing an optimal hair removal map of the user's skin. A spatial location of the optimal hair removal map may be associated with an optimal hair removal technique. The optimal hair removal technique may be determined based on at least one of: pre-hair removal skin parameter data; historical data for the user; and predetermined knowledge regarding hair removal. The method may further comprise determining the hair removal instruction for the spatial location based on the optimal hair removal map.

In some embodiments, the method comprises determining, in real-time, the position of the hair removal unit relative to the user's skin. In some embodiments, the method comprises determining, in real-time, the interaction between the user's skin and the hair removal unit. In some embodiments, the method comprises determining, in real-time, the skin parameter. The method may further comprise determining a real-time hair removal instruction for the user based on at least one of: the position; the interaction; the skin parameter; historical hair removal performance data for the user; and pre-determined hair removal performance data.

In some embodiments, the method comprises causing the user interface to provide, in real-time, the hair removal instruction for the user.

In some embodiments, the historical hair removal performance data for the user comprises at least one of: user skin type; user skin condition; pressure applied between the hair removal unit and the user's skin; user hair removal behavior; visible skin irritation; hair removal results; hair removal unit motion and hair removal unit operational performance. The historical hair removal performance data may be determined from at least one previous user hair removal session. The pre-determined hair removal performance data may comprise knowledge acquired from other users and/or clinical data regarding at least one of: skin type; skin condition; pressure applied between the hair removal unit and the other user's skin; hair removal behavior; visible skin irritation; hair removal results; hair removal unit motion and hair removal unit operational performance. A recommended hair removal instruction for the user can be determined in order to provide improved hair removal experience as compared to a previous user hair removal session. The recommended hair removal instruction may be based on at least one the historical hair removal performance data for the user and the pre-determined hair removal performance data.

In some embodiments, the hair removal instruction is configured to provide a personalized recommendation for the user regarding at least one of: pressure to apply between the hair removal unit and the user's skin; hair removal unit positioning relative to the user's skin and hair removal unit motion. The method may further comprise causing the user interface to provide the hair removal instruction for the user based on whether or not the user has deviated from a previously-recommended hair removal instruction.

In some embodiments, determining the position of the hair removal unit relative to the user's skin comprises acquiring at least one of: imaging data of the user's skin and the hair removal unit; and motion data from a sensor on-board the hair removal unit. The position of the hair removal unit relative to the user's skin may comprise at least one of: a position of a hair removal device of the hair removal unit on the user's skin; and an orientation of the hair removal device relative to the user's skin.

In a second aspect, apparatus comprising processing circuitry is described. The processing circuitry comprises an obtaining module, a determining module and a user instruction module. The obtaining module is configured to obtain an indication comprising a skin parameter of a user. The indication further comprises an interaction between the user's skin and a hair removal unit. The determining module is configured to determine a position of the hair removal unit relative to the user's skin. The determining module is further configured to determine a hair removal instruction for the user based on the indication and the position. The user instruction module is configured to cause a user interface to provide the hair removal instruction for the user.

In some embodiments, the apparatus further comprises at least one of: an imaging device and the user interface. The imaging device may be for acquiring imaging data of the user's skin and the hair removal unit.

In a third aspect, a tangible machine-readable medium is described. The tangible machine-readable medium stores instructions which, when executed by at least one processor, cause the at least one processor to obtain an indication comprising a skin parameter of a user. The indication further comprises an interaction between the user's skin and a hair removal unit. The instructions further cause the at least one processor to determine a position of the hair removal unit relative to the user's skin. The instructions further cause the at least one processor to determine a hair removal instruction for the user based on the indication and the position. The instructions further cause the at least one processor to cause a user interface to provide the hair removal instruction for the user.

These and other aspects of the invention will be apparent from and elucidated with reference to the embodiment(s) described hereinafter.

### BRIEF DESCRIPTION OF DRAWINGS

Exemplary embodiments of the invention will now be described, by way of embodiment only, with reference to the following drawings, in which:
Figure 1 refers to a method of providing hair removal instructions for a user according to an embodiment;
Figure 2 is a schematic drawing of a system for providing hair removal instructions for a user according to an embodiment;
Figure 3 refers to a method of providing hair removal instructions for a user according to an embodiment;
Figure 4 is a schematic drawing of an apparatus for providing hair removal instructions according to an embodiment;
Figure 5 is a schematic drawing of an apparatus for providing hair removal instructions according to an embodiment; and
Figure 6 is a schematic drawing of a machine-readable medium for providing hair removal instructions according to an embodiment.

### DETAILED DESCRIPTION OF THE EMBODIMENTS

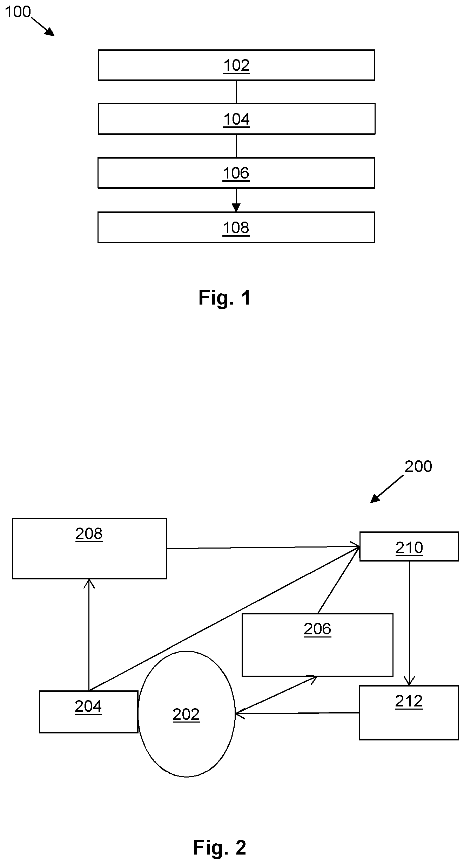

Figure 1 shows a method 100 (e.g., a computer-implemented method) of providing hair removal instructions (e.g., guidance) for a user. As will be described in more detail herein, the method 100 may allow the user to improve and/or optimize hair removal results and/or reduce skin irritation due to hair removal.

The method 100 comprises, at block 102, obtaining an indication. The indication comprises a skin parameter of a user. The skin parameter may refer to a characteristic of the skin that may affect hair removal and/or be affected by hair removal. A spatial location on the user's skin may be associated with the skin parameter. Accordingly, a map of the user's skin may comprise a plurality of spatial locations where each spatial location has an associated skin parameter. A plurality of skin parameters may be associated with each spatial location.

The skin parameter may refer to, for example, skin type, skin health status, skin moisture, skin roughness, after-hair removal irritation (e.g., skin redness) of the user and/or any skin characteristic associated with the user's skin. The skin parameter may be indicative of certain information regarding the user's skin. The information may comprise a calculated or estimated value indicating, for example, a skin irritation level where different values may indicative a different level of skin irritation. Where a plurality of skin parameters are associated with a spatial location on the skin, different skin parameters may be associated with that spatial location. At least one of the skin parameters may provide certain information to facilitate e.g., improved hair removal results and/or a reduction in skin irritation from hair removal.

The obtained indication further comprises an interaction between the user's skin and a hair removal unit. For example, the interaction may comprise a pressure applied by the user on the user's skin by the hair removal unit.

The method 100 further comprises, at block 104, determining a position of the hair removal unit relative to the user's skin.

The method 100 may allow the position of the hair removal unit to be tracked in relation to the user's skin. A more detailed description of the hair removal unit position determination is provided below.

The method 100 further comprises, at block 106, determining a hair removal instruction for the user based on the indication and the position.

The method 100 may take into account certain information derived from the indication and/or the position of the hair removal unit to determine the hair removal instruction. As will be described in more detail below, the hair removal instruction may be used to provide guidance for the user in terms of how to remove hair from their skin using the hair removal unit. For example, the hair removal instruction may determine that the user should apply more or less pressure using the hair removal unit. Additionally or alternatively, the hair removal instruction may determine guidance in terms of the direction, hair removal unit orientation and/or speed with which the user is to use the hair removal unit. Additionally or alternatively, the method 100 may determine a hair removal instruction to provide to the user that is indicative of a skin and/or hair treatment regime, hair removal unit charge level and any other factor which may affect whether or not the hair removal session provides improved/optimal hair removal results and/or reduces skin irritation.

Determining the position of the hair removal unit relative to the user's skin may provide certain information which may be used to determine the hair removal instruction. For example, a determination may be made that the hair removal unit is at or about to arrive at a certain spatial location on the user's skin. A determination may be made, based on the indication and/or the position, regarding a recommended hair removal unit action/technique for that spatial location on the skin and/or the next/predicted spatial location on the skin.

The method 100 further comprises, at block 108, causing a user interface to provide the hair removal instruction for the user.

Once the hair removal instruction has been determined, the hair removal instruction may be provided in an appropriate format to enable the user to identity the hair removal instruction and attempt to follow the hair removal instruction. The hair removal instruction may be provided in any appropriate format for the user (e.g., via a visual and/or audible format). The user interface may comprise a device capable of providing the hair removal instruction for the user in a visual and/or audible manner. The user interface may be provided by a user equipment such as a mobile phone, tablet, mirror, smart device or any other device capably of conveying visual and/or audible instructions. For example, the user may possess a user equipment capable of providing a visualization of the user's skin (e.g., via a graphical user interface such as a screen) and corresponding hair removal instructions. For example, an arrow, moving indicator or other hair removal instruction may be visualized on the screen, which the user can interpret and follow. Additionally or alternatively, the user equipment may provide an audible hair removal instruction. For example, if too much or too little pressure is applied by the hair removal unit, an audible warning such as a beep or verbal instruction may be played for the user. Any combination of visual and/or audible hair removal instructions may be provided by the user interface.

Accordingly, the method 100 may provide a user with hair removal guidance, which may help the user to achieve improved and/or optimal hair removal results and/or reduce skin irritation due to hair removal. Thus, a user may be made aware of the optimal hair removal technique for reducing skin irritation from hair removal. By obtaining the indication (e.g. at block 102) and/or the position of the hair removal unit (e.g., at block 104), the hair removal instruction may be tailored to the user's individual needs e.g., to provide improved hair removal results and/or reduce skin irritation.

The method 100 may enable the user to be trained so that their future hair removal sessions provide improved/optimal results and/or reduce skin irritation. In some embodiments, data from different hair removal sessions may be compared to determine whether or not hair removal performance could be improved and/or whether or not skin irritation could be reduced. The hair removal instruction may take into account such a comparison e.g., to learn how the user could improve their hair removal results.

Figure 2 shows a system 200 for implementing certain methods described herein. In the system 200, a user 202 removes hair from their skin (e.g., from their face or another part of their body) with a hair removal unit 204. The hair removal unit 204 may comprise an electric shaver (e.g., a motorized rotary blade or a foil-based razor), an epilator, a manual razor, smart razor or indeed any type of hair removal unit capable of removing hair from skin whether by cutting, pulling or otherwise removing at least a portion of the hair from the skin. Any reference to shaving may refer to any form of hair removal e.g., by any type of hair removal unit.

The hair removal unit 204 may comprise at least one sensor for determining the interaction between the user's skin and the hair removal unit 204. For example, the hair removal unit 204 may comprise a pressure sensor for measuring contact pressure between the user's skin and the hair removal unit 204.

A user measurement unit 206 of the system 200 is configured to determine the indication of the skin parameter and/or an indication of any other parameters described herein (for example, a hair parameter). The user measurement unit 206 may acquire information relating to the skin parameter and/or any other parameters (e.g., from imaging data acquired by a user equipment) in order to determine the indication. For example, the imaging data may indicate that a hair removal session has caused some skin irritation, which may be apparent by comparing the redness in the skin between different images. Thus, the user measurement unit 206 may perform measurements which can be used to determine the indication of the skin parameter.

The user measurement unit 206 may map the skin in order to determine the indication of the skin parameter (and/or any other parameters) for different spatial locations on the user's skin.

The user measurement unit 206 may perform processing (e.g., using on-board processing circuitry of a user equipment) to determine the skin parameter and/or any other parameters described herein. Additionally or alternatively, the user measurement unit 206 may send its acquired data to an online service so that the online service may determine the skin parameter and/or any other parameters. Further the processing may facilitate mapping of the user's skin such that a spatial location is associated with a certain skin parameter and/or any other parameter described herein.

In some embodiments, the user measurement unit 206 is configured to map certain relevant skin and/or hair parameters such as skin moisture, skin roughness, hair growth orientation, hair length, hair density, post-hair removal irritation/redness of the user.

The user measurement unit 206 may be configured to cause an on-board sensor of the user equipment (e.g., an imaging device such as a camera) to acquire data such as imaging data to be used (e.g., by the user measurement unit 206 itself or an online service) to determine the skin parameter and/or any other parameter.

The position of the hair removal unit 204 relative to the user's skin may be determined by a hair removal unit localization unit 208. The position of the hair removal unit 204 may refer to or be indicative of a position of a hair removal device (e.g., a blade) of the hair removal unit 204 on the user's skin. Additionally or alternatively, the position of the hair removal unit 204 may refer to or be indicative of an orientation of the hair removal device relative to the user's skin. The hair removal unit 204 may comprise the hair removal device and other components such as a handle. While performing hair removal, the position of the hair removal device itself may be used to determine the hair removal instruction. Thus, the hair removal unit localization unit 208 may determine the position of the hair removal device itself, which may provide accurate information regarding the spatial location on the user's skin where the hair removal device (e.g., the blade) is located. Additionally or alternatively, the position of any other part of the hair removal unit 204 may be determined, which may infer or be used to determine the position of the hair removal device relative to the user's skin.

The hair removal unit localization unit 208 may be implemented by any user equipment (e.g., a smart device) or other user device depending on the procedure used to determine the position of the hair removal unit 204. For example, the hair removal unit 204 itself may comprise an on-board sensor such as a motion-sensitive detector (e.g., accelerometer) and/or an imaging device to determine its position relative to the user's skin.

Additionally or alternatively, the user equipment (e.g., a smart device) may comprise or at least partially implement the hair removal localization unit 208. For example, the user equipment may comprise an imaging device such as a camera for acquiring imaging data, which can be used to track the position of the hair removal unit 204 and/or the user's hand relative to the user's skin. The imaging data may be processed by on-board processing circuitry of the user equipment and/or may be communicated to an online service or other processing apparatus to be processed. The tracking of the position of the hair removal unit 204 relative to the user's skin may, for example, involve a machine vision-based tracking procedure. The tracking procedure may also take into account the user's skin using a skin recognition algorithm. For example, if tracking the hair removal unit 204 on the user's face, a facial recognition algorithm in combination with a tracking algorithm may be used to determine where, on the user's face, the hair removal unit 204 is positioned.

The system 200 comprises a processing unit 210 for implementing certain methods described herein, such as the method 100 of Figure 1. The processing unit 210 comprises processing circuitry for implementing the method. In this embodiment, the processing unit 210 obtains the indication comprising the skin parameter and/or any other parameter of the user from the user measurement unit 206 (e.g., in accordance with block 102 of the method 100). The processing unit 210 further obtains the indication comprising the interaction between the user's skin and the hair removal unit 204 from the hair removal unit 204 (e.g., in accordance with block 104 of the method 100). The processing unit 210 determines the position of the hair removal unit 204 relative to the user's skin based on data provided by the hair removal unit localization unit 208 (e.g., in accordance with block 106 of the method 100).

The processing unit 210 determines a hair removal instruction for the user 202 based on the indication and the position (e.g., in accordance with block 106 of the method 100). In some embodiments, the processing unit 210 may determine a map of optimal hair removal instructions for at least one spatial location on the user's skin. For example, the hair removal instruction may be indicative of at least one of: an applied pressure, hair removal unit 204 motion direction, hair removal unit 204 motion speed that is recommended for the user 202 based on at least one of: the skin parameter (e.g., as provided in a skin measurement map), any other parameters as described herein; and the position of the hair removal unit 204 relative to the user's skin.

The processing unit 210 causes a user interface 212 of the system 200 to provide the hair removal instruction to the user (e.g., in accordance with block 108 of the method 100). In some embodiments, the user interface 212 comprises a display for visualization of the recommended hair removal instruction mapped on to a skin and/or hair parameter map to provide real-time guidance (e.g., visual guidance) to the user for hair removal that may lead to improved and/or optimal hair removal results and/or reduced/minimized skin irritation.

The user interface 212 may be provided by a user equipment that is the same as or different to the user equipment or user device providing the user measurement unit 206 and/or the hair removal unit localization unit 208. For example, a smart phone or other smart device may perform imaging via its on-board camera to obtain the indication and the position. Further, a display screen and/or speaker of the smart device may be configured to provide the hair removal instruction (e.g., in a format appropriate for the user to interpret).

In some embodiments, the processing unit 210 further comprises or can access a memory unit for storing at least one of: certain measurements (such as obtained by the hair removal unit 204, user measurement unit 206 and/or hair removal unit localization unit 208), a skin and/or hair parameter map (e.g., comprising a skin, hair and/or other parameter for a corresponding spatial location on the skin), a derived skin parameter and/or any other parameter, hair removal unit position relative to the user's skin, a map of hair removal unit 204 usage (e.g., previous usage) and a map of optimal hair removal unit 204 instructions.

In some embodiments, the processing unit 210 may calculate an optimal hair removal unit 204 configuration. The hair removal unit 204 configuration may refer to, for example, a blade speed, blade rotation speed, cutting force and/or power for the hair removal unit 204. The processing unit 210 may provide feedback to the hair removal unit 204 such that the hair removal unit 204 adapts in real-time for optimal hair removal. The calculation of the optimal hair removal unit 204 configuration may be provided in addition to certain methods described herein or may replace certain blocks of certain methods. For example, the calculation of the optimal hair removal unit 204 configuration may be provided in addition to the blocks of the method 100. Alternatively, blocks 106 and 108 of the method 100 may be omitted and the calculation of the optimal hair removal unit 204 configuration may be implemented in combination with blocks 102 and 104 of the method 100. Alternatively, blocks 104, 106 and 108 of the method 100 may be omitted and the calculation of the optimal hair removal unit 204 configuration may be implemented in combination with block 102 of the method 100.

The processing unit 210 for implementing certain methods described herein may be provided by a user equipment such as described above. Alternatively, the processing unit 210 may be provided by an online service (e.g., at a server or cloud-based service).

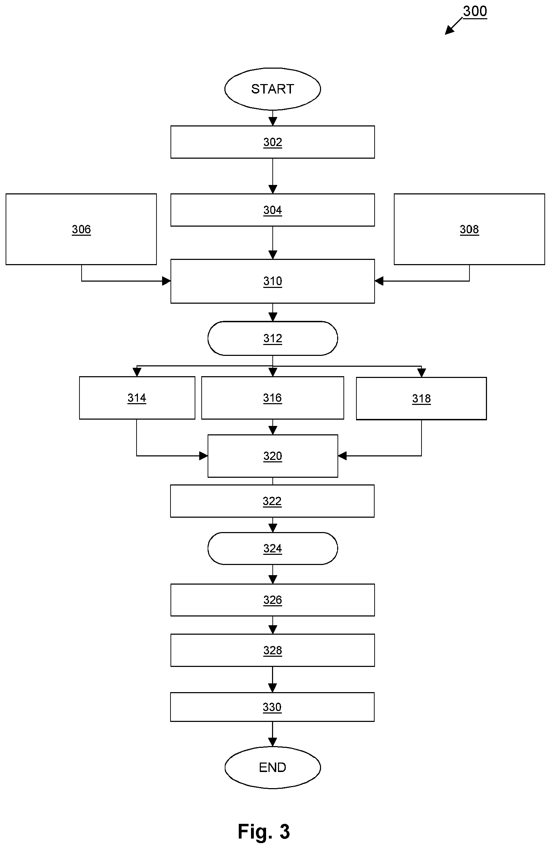

Figure 3 shows a method 300 (e.g., a computer-implemented method) of providing hair removal instructions for a user. The method 300 may be implemented by processing circuitry such as provided by the processing unit 210 of Figure 2 or any other processing apparatus or circuitry described herein. As will be described in more detail herein, the method 300 may allow the user to improve and/or optimize hair removal results and/or reduce skin irritation due to hair removal. The method 300 may comprise certain blocks corresponding to certain blocks of the method 100. Certain blocks of the method 300 may be omitted and/or modified.

In some embodiments, a hair removal unit such as a shaver may be communicatively coupled to a smart device, e.g. smartphone loaded with a Real Time Shaving Guidance application, or 'app', to assist a user with hair removal. The hair removal unit may be used for shaving/removing facial hair and/or for removing hair from any other part of the body.

The method 300 comprises, at block 302, acquiring imaging data (e.g., at least one image) of the user's skin. For example, the block 302 may cause an imaging device (e.g., of a user equipment) to obtain the imaging data. In this block 302, the imaging data is acquired prior to a user hair removal session.

Based on the imaging data, the method 300 comprises, at block 304, determining certain data regarding a skin and/or hair parameter associated with the user. The data regarding the skin and/or hair parameter may be referred to as pre-hair removal skin parameter data (i.e., the data may relate to the skin and/or hair parameter). In some embodiments, an algorithm may determine the skin and/or hair parameter based on a machine learning model which has been trained to identity and/or classify certain skin parameters (e.g., skin redness, for example) from the imaging data.

A corresponding skin and/or hair parameter map may be generated by the processing circuitry based on the skin and/or hair parameter data. The skin and/or hair parameter map may comprise at least one skin and/or hair parameter (and/or any other parameter) associated with at least one spatial location of the user's skin.

A user may initially capture an image of their skin (e.g., their face) using their smart device to obtain a baseline skin parameter map (e.g., a baseline facial skin map). This can be done via certain facial tracking techniques (e.g., based on machine learning or another algorithm). During this imaging data acquisition, the present skin and/or hair conditions (e.g., length and/or type of hair) for each spatial location may be determined and recorded in a memory for future use. This image capture and present skin/hair condition determination procedure may be performed before each hair removal session and/or may be performed before the first (i.e., first ever) hair removal session with the hair removal unit.

In some embodiments, the baseline skin parameter map may comprise any relevant skin and/or hair parameters (e.g., skin moisture, skin roughness, hair growth orientation, hair length, hair density, post-hair removal irritation/redness of the user).

The method 300 further comprises, at block 306, providing or accessing historical data for the user (e.g., from a memory such as the memory unit referred to in relation to Figure 2).

A previous hair removal session may have yielded certain data regarding hair removal performance and/or at least one skin and/or hair parameter associated with the user. For example, if the previous hair removal session caused skin irritation, this may be reflected by the corresponding skin and/or hair parameter for the spatial location(s) affected by the skin irritation. The historical data may comprise or be used to calculate a comparison of an outcome of a hair removal session (e.g., a comparison of the post-hair removal skin parameter data with the pre-hair removal skin parameter data).

In some embodiments, the historical data may comprise or be referred to as a post-hair removal skin parameter map (i.e., the historical data may relate to the skin and/or hair parameter). The post-hair removal skin parameter map may have been obtained previously after a previous hair removal session. The post-hair removal skin parameter map may comprise the comparison of a skin and/or hair parameter map obtained before and after the hair removal session.

The method comprises, at block 308, providing or accessing predetermined knowledge regarding hair removal (which knowledge may be stored in a memory e.g., of an online service or of a user equipment). For example, the predetermined knowledge may comprise general (e.g., clinical) knowledge on hair removal techniques and/or the skin-hair interaction. Such knowledge may comprise, for example, at least one of: an optimal hair removal unit pressure to apply on the skin (e.g., for certain skin types and/or position of the hair removal unit); optimal hair removal unit speed across the user's skin; optimal hair removal unit direction and/or motion pattern for certain spatial locations on the skin and/or hair lengths/types.

The predetermined knowledge may be used for providing an initial recommendation on the hair removal technique. In some embodiments, further recommendations may be personalized based on data obtained from subsequent hair removal sessions. Thus, the method 300 comprises, at block 310, generating an optimal hair removal map of the user's skin. A spatial location of the optimal hair removal map may be associated with an optimal hair removal technique that is determined based on at least one of: historical data for the user (e.g., from block 304); pre-hair removal skin parameter data (e.g., from block 306) and predetermined knowledge regarding hair removal (e.g., from block 308).

In some embodiments, the optimal hair removal map may be stored in a memory (e.g., of a user equipment or an online service), for example, to allow the optimal hair removal map to be accessed during or after a hair removal session.

The spatial location may be associated with at least one of: a skin parameter and a hair parameter for the user's skin at that spatial location. For example, the optimal hair removal map may provide an indication of the skin and/or hair parameter at the spatial location. The indication of the skin and/or hair parameter provided by the optimal hair removal map may be referred to as pre-hair removal skin and/or hair parameter data.

In some embodiments, the method 300 comprises accessing the optimal hair removal map of the user's skin (e.g., as generated according to block 310); and determining the hair removal instruction for the spatial location based on the optimal hair removal map. For example, the method 300 comprises, at block 312, starting a hair removal session in which the optimal hair removal map is accessed.

In some embodiments, obtaining the indication (e.g., in accordance with block 102 of the method 100) comprising the skin parameter of the user comprises accessing skin parameter data for the user determined based on imaging data of the user's skin. For example, the imaging data of the user's skin may refer to the imaging data acquired at block 302 of the method 300. As described above, the optimal hair removal map may provide the indication of the skin parameter. Thus, obtaining the indication comprising the skin parameter of the user may comprise accessing the optimal hair removal map (e.g., as described in relation to blocks 310/312 of the method 300).

Additionally or alternatively, the imaging data of the user's skin may refer to imaging data acquired at another block of the method 300, which may be used to provide the indication comprising the skin parameter of the user (for example, in real-time, as will be described in more detail herein).

Before or during a hair removal session, the optimal hair removal map may be accessed in order to allow determination of a hair removal instruction for the user based on the indication comprising the skin parameter of the user (e.g., as referred to in block 106 of the method 100). As will be described in more detail below, the method 300 may use information derived from the optimal hair removal map in conjunction with other information obtained during the hair removal session in order to determine the hair removal instruction.

The method 300 comprises, at block 314, determining, in real-time, the position of the hair removal unit relative to the user's skin. The data for determining the position of the hair removal unit in block 314 may be obtained by the hair removal unit localization unit 208 described in relation to Figure 2. This data may be obtained from at least one of: imaging data acquired from an imaging device for acquiring images of the skin; and an on-board sensor of the hair removal unit 304. The determination of the position of the hair removal unit may be determined from this data (e.g., using the processing unit 210 of Figure 2).

In some embodiments, determining the position of the hair removal unit relative to the user's skin comprises acquiring at least one of: imaging data of the user's skin and the hair removal unit; and motion data from a sensor on-board the hair removal unit.

The position of the hair removal unit relative to the user's skin may comprise at least one of: a position of a hair removal device of the hair removal unit on the user's skin; and an orientation of the hair removal device relative to the user's skin.

Positioning data may be obtained from the imaging data and/or the on-board sensor. This positioning data may be used to track the position of the hair removal unit relative to the user's skin as the user moves the hair removal unit across their skin.

In the example of the app give above, during the hair removal session, the app may determine the real-time motion (e.g., position and/or orientation) of the hair removal unit relative to the user's skin, using a series of images captured by a camera of the user equipment. This determination of the real-time motion can be performed by tracking the hair removal unit and/or the user's hand within the image series, for example, using a computer vision algorithm. The determination may be supported by motion and/or orientation tracking within the hair removal unit itself (e.g., using an on-board sensor of the hair removal unit).

The method 300 further comprises, at block 316, determining, in real-time, the interaction between the user's skin and the hair removal unit. As mentioned previously, the hair removal unit 204 may comprise at least one sensor for determining the interaction (e.g., applied pressure) between the user's skin and the hair removal unit 204. In some embodiments, data for determining the interaction may be obtained from the hair removal unit 204.

For example, during a hair removal session, the applied pressure may be recorded in real-time and may be linked to the position of the hair removal unit.

In some embodiments, the amount of hair cut/removed may be recorded or inferred. For example, the amount of hair cut/removed may be determined from at least one of: a sound analysis (e.g., using a microphone of a user equipment or of the hair removal unit itself to determine how many hairs are being cut or removed); a motor resistance observed by a hair removal device (e.g., motorized blade) of the hair removal unit (i.e., the motor resistance may be affected by pressure on skin and/or number of hairs cut/removed); and a computer vision analysis (e.g., using imaging data obtained from a camera of the user equipment) of the results obtained during the hair removal session.

The method 300 comprises, at block 318, determining, in real-time, the skin parameter. As mentioned above, the indication comprising the skin parameter of the user may be determined from imaging data acquired before the hair removal session. In block 318, the skin parameter may be determined from imaging data (e.g., at least one image) acquired during the hair removal session. For example, the imaging data may be acquired by an imaging device of a user equipment and this imaging data may be processed in order to provide the indication comprising the skin parameter of the user (e.g., in a similar manner to block 304 determining certain data regarding the skin parameter associated with the user).

In some embodiments, block 318 may cause the imaging device to acquire the imaging data during the user hair removal session, to determine present skin parameter data. The skin parameter of the user may be based on a comparison between the pre-hair removal skin parameter data (e.g., as referred to in block 310) and the present skin parameter data.

The method 300 comprises, at block 320, determining a real-time hair removal instruction for the user based on at least one of: the position; the interaction; the skin parameter; historical hair removal performance data for the user; and pre-determined hair removal performance data. Thus, at least one of blocks 314, 316 and 318 may be implemented in order to determine the real-time hair removal instruction. For example, the real-time hair removal instruction may provide at least one of: a recommended pressure, hair removal unit motion direction and/or pattern and/or hair removal unit motion speed which has been calculated for each position on the skin and/or hair parameter map.

The historical hair removal performance data for the user may comprise at least one of: user skin type; user skin condition; pressure applied between the hair removal unit and the user's skin; user hair removal behavior; visible skin irritation (e.g., skin redness); hair removal results (e.g., hair cutting effectiveness); hair removal unit motion (e.g., direction and type of movement) and hair removal unit operational performance (e.g., battery level, cutting speed). The historical hair removal performance data may be determined from at least one previous user hair removal session.

This historical hair removal performance data may be an example of the historical data provided at block 306 of the method 300. The term 'visible' in relation to the skin irritation may refer to whether or not the skin irritation is visible to a machine vision system or any system capable of detecting skin irritation, whether visible to the human eye, or not.

The pre-determined hair removal performance data may comprise knowledge acquired from other users and/or clinical data regarding at least one of: skin type; skin condition; pressure applied between the hair removal unit and the other user's skin; hair removal behavior; visible skin irritation; hair removal results; hair removal unit motion and hair removal unit operational performance.

This knowledge may be used to determine a recommended hair removal instruction for the user in order to provide improved hair removal experience (e.g., more efficient cutting, less time, less skin irritation) as compared to a previous user hair removal session. This pre-determined hair removal performance data may be an example of the pre-determined knowledge regarding hair removal provided at block 308 of the method 300.

The method 300 further comprises, at block 322, comprising causing a user interface (e.g., the user interface 212 of Figure 2) to provide, in real-time, the real-time hair removal instruction for the user.

At block 324 of the method 300, the user ends their hair removal session. Certain blocks described below may be used to evaluate the results of the hair removal session, which may provide certain information that can be used in a subsequent hair removal session (e.g., the information may be stored in a memory (e.g., of a user equipment or an online service) so as to be provided at block 306).

The method 300 comprises, at block 326, causing the imaging device to acquire the imaging data (i.e., after the user hair removal session), to determine post-hair removal skin parameter data. At block 328 of the method 300, skin and/or hair parameter data is generated (which may be referred to as post-hair removal skin parameter data). This skin and/or hair parameter data may be used to generate a skin and/or hair parameter map. The post-hair removal skin parameter data may relate to a skin and/or hair parameter and/or any other parameter.

The method 300 further comprises, at block 330, determining a skin and/or hair parameter map for the user based on a comparison between the pre-hair removal and present and/or post-hair removal skin parameter data. The comparison may be made between a map derived from the present and/or post-hair removal skin parameter data and the baseline skin parameter map.

In some embodiments, the skin parameter comprises a visible skin irritation indicator (e.g., skin redness). The skin parameter may be based on whether or not the comparison (e.g., at block 330) identifies any change in the visible skin irritation indicator between the pre-hair removal and present and/or post-hair removal skin parameter data.

For example, after the first hair removal session, the skin redness is measured and a map of the redness is saved (e.g., the map may correspond to the skin parameter map). This measurement may be performed by analyzing data such as imaging data acquired from a camera of a user equipment and/or from a separate skin analysis device. The analysis may comprise a comparison with the image(s) captured before the hair removal session began and/or use data provided by the separate skin analysis device. The separate skin analysis device may refer to any other device capable of measuring a certain property of the skin such as hydration, gloss/oiliness, spots and redness, among other properties. Certain examples of such skin analysis devices may illuminate the skin with radiation (e.g., ultraviolet, visible and/or infrared) and detect characteristics (such as a change in spectral content and/or intensity) from the radiation reflected by the skin.

Additionally or alternatively, certain data such as the recorded hair removal unit applied pressure, skin redness, hair removal results and/or the hair removal unit motion data may be processed to identify hair removal actions/techniques related to a position on the skin where those actions/techniques resulted in optimal, sub-optimal or poor hair removal results.

For example, the data acquired at certain blocks (e.g., blocks 302, 314, 316, 318, 326) and analyzed (e.g., at blocks 310, 328, 330) may provide an indication that can be used to update the hair removal instruction. In an example, too much pressure applied by the hair removal unit on the skin may result in skin irritation and/or suboptimal hair cutting. In another example, insufficient skin contact (e.g., including suboptimal orientation of the hair removal unit in relation to the skin) may result in sub-optimal hair cutting and/or hair pulling. In another example, a suboptimal direction or motion pattern (e.g., straight vs circular) of the hair removal unit may result in suboptimal hair cutting. In another example, suboptimal hair removal unit motion speeds (e.g., too fast or too slow) may result in skin irritation, suboptimal hair cutting and/or shaving inefficiency. In another example, too many passes of the hair removal unit over a particular spatial location of the skin may be a result of suboptimal hair cutting, resulting in skin irritation and/or shaving inefficiency. In another example, other suboptimal hair removal techniques may be identified that can be improved by different user and/or hair removal unit behavior. Any combination of these and other examples may be identified from the acquired data and used to recommend a technique (e.g., hair removal instruction) to the user which may result in improved and/or optimal hair removal and/or reduced skin irritation.

During a subsequent hair removal session (e.g., after the first or initial hair removal session), certain blocks of the method 300 may be implemented. For example, the real-time motion (e.g., position and/or orientation) of the hair removal unit relative to the skin may be determined using a series of images captured by the camera (e.g., in accordance with block 318). In some embodiments, other parameters such as the skin and/or hair parameter may be determined and/or other sensors may be used as well to provide data which can be used to determine the recommendation for the user.

For example, based on the real-time position of the hair removal unit, the recommended hair removal unit pressure, direction and speed may be visually shown to the user in real-time. In a further example, the recommended hair removal guidance may be shown (e.g., by a display of a user equipment) in relation to the actual shaving behavior of the user, which may provide direct cues to the user such as indicating: an increase or decrease of applied pressure, an increase or decrease in the motion direction and/or a certain motion pattern (e.g., straight or circular motion and/or a different diameter of circular motion) and/or an increase or decrease in motion speed.

During the subsequent hair removal session, the applied hair removal pressure (and/or other parameters) may be recorded in real-time and linked to the position of the hair removal unit.

After the subsequent hair removal session, the skin irritation (e.g., skin redness) may be measured again and a map of the skin irritation may be saved in a memory (and used to update the hair removal instruction).

The recorded pressure, skin irritation, position data and/or any previous recommendations may be processed, to calculate personalized recommendations. These calculated personalized recommendations may be based on the level of adherence to the guidance indicated by the cues described above.

Additionally or alternatively, these recommendations may be based on the results in terms of skin irritation and/or hair removal efficiency. In some cases, perfect adherence by the user can still lead to suboptimal results, which may indicate that the general knowledge may not apply to this user and may need to be personalized for the user by learning from data acquired from the user's hair removal session(s).

Once the personalized recommendations have been calculated, the recommended pressure, motion direction and/or motion speed may be recalculated for each position in the skin and/or hair parameter map for the next hair removal session.

In some embodiments, the hair removal instruction is configured to provide a personalized recommendation for the user regarding at least one of: pressure to apply between the hair removal unit and the user's skin; hair removal unit positioning (e.g., including orientation of the hair removal unit) relative to the user's skin and hair removal unit motion (e.g., including direction, speed and motion pattern of the hair removal unit). In other words, methods described herein may obtain information such as the skin and/or hair parameter for the user and thereby determine the personalized recommendation.

Certain embodiments described herein refer to a user's skin parameter, an indication of which is obtained by certain methods described herein. In some embodiments, the indication further comprises a hair parameter of the user. Thus, certain methods described herein may further comprise determining the hair removal instruction taking into account the hair parameter. In other words, the hair removal instruction may be based on an analysis of the skin and/or hair parameter acquired from imaging data, which may have been obtained prior to a hair removal session (e.g., at block 302 or from a previous hair removal session's block 326) or during a hair removal session (e.g., at block 318).

In some embodiments, the method 300 may further comprise causing the user interface to provide the hair removal instruction for the user based on whether or not the user has deviated from a previously-recommended hair removal instruction. For example, during a hair removal session, a user may deviate from the recommended hair removal instruction. The method 300 may then update the hair removal instruction to accommodate/correct for the user's deviation.

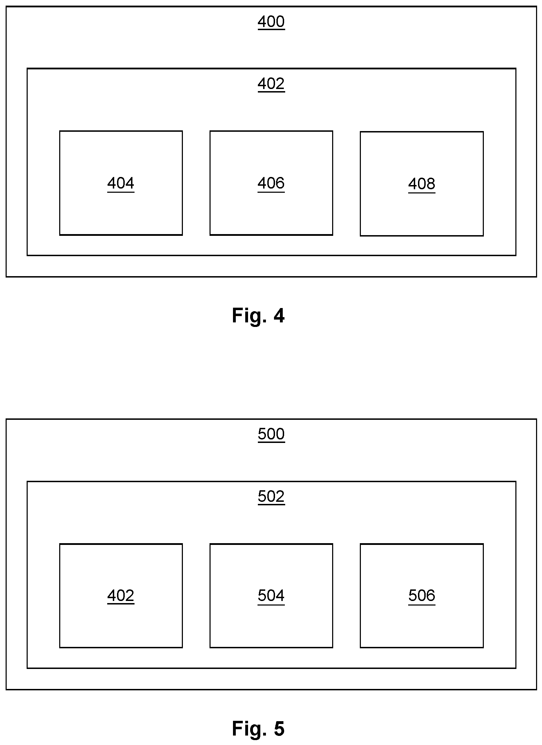

Figure 4 shows an apparatus 400, which may be used for implementing certain methods described herein such as the methods 100, 300. The apparatus 400 comprises processing circuitry 402. The processing circuitry 402 may correspond to the processing circuitry of the processing unit 210 described in relation to Figure 2. In this embodiment, the processing circuitry 402 comprises an obtaining module 404. The obtaining module 404 is configured to obtain an indication comprising a skin parameter of a user; and an interaction between the user's skin and a hair removal unit (such as described in relation to block 102 of the method 100).

The processing circuitry 402 further comprises a determining module 406. The determining module 406 is configured to determine a position of the hair removal unit relative to the user's skin (such as described in relation to block 104 of the method 100). The determining module 406 is further configured to determine a hair removal instruction for the user based on the indication and the position (such as described in relation to block 106 of the method 100).

The processing circuitry 402 further comprises a user instruction module 408. The user instruction module 408 is configured to cause a user interface to provide the hair removal instruction for the user (such as described in relation to block 108 of the method 100).

Figure 5 shows an apparatus 500, which may be used for implementing certain methods described herein such as the methods 100, 300. The apparatus 500 comprises processing circuitry 502. The processing circuitry 502 comprises the processing circuitry 402 of the apparatus 400 of Figure 4.

In some embodiments, the apparatus 500 further comprises an imaging device 504 such as a camera of a user equipment for acquiring imaging data of the user's skin and the hair removal unit (e.g., hair removal unit 204 of Figure 2).

In some embodiments, the apparatus 500 further comprises the user interface 506 (e.g., as referred to in the user instruction module 408) of a user equipment.

In some embodiments, the apparatus 500 comprises both the imaging device 504 and the user interface 506.

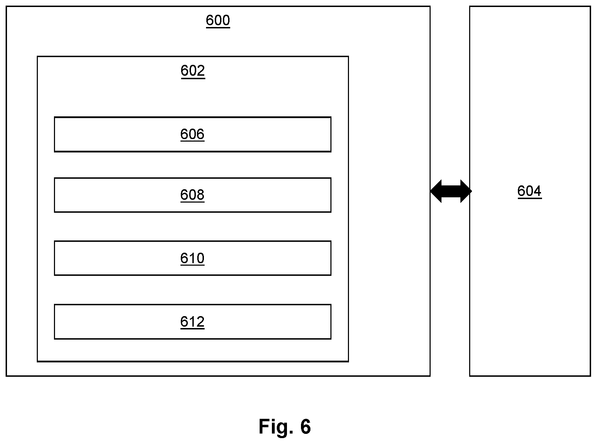

Figure 6 shows a tangible machine-readable medium 600 storing instructions 602 which, when executed by at least one processor 604, cause the at least one processor 604 to implement certain methods described herein (such as the methods 100, 300).

The instructions 602 comprise instructions 606 that cause the at least one processor 604 to obtain an indication comprising a skin parameter of a user; and an interaction between the user's skin and a hair removal unit (such as described in relation to block 102 of the method 100).

The instructions 602 further comprise instructions 608 that cause the at least one processor 604 to determine a position of the hair removal unit relative to the user's skin (such as described in relation to block 104 of the method 100).

The instructions 602 further comprise instructions 610 that cause the at least one processor 604 to determine a hair removal instruction for the user based on the indication and the position (such as described in relation to block 106 of the method 100).

The instructions 602 further comprise instructions 612 that cause the at least one processor 604 to cause a user interface to provide the hair removal instruction for the user (such as described in relation to block 108 of the method 100).

One or more features described in one embodiment may be combined with or replace features described in another embodiment. For example, the methods 100 and 300 of Figures 1 and 3 may be modified based on features described in relation to the system 200 and apparatus 400, 500 of Figures 2, 4 and 5, and vice versa.

In some embodiments, certain methods described herein may be implemented by processing circuitry of a user equipment such as a mobile phone, tablet, mirror, smart device or any other device. In some embodiments, certain methods described herein may be implemented by processing circuitry of an online service such as provided by a server or cloud-based service. In some embodiments, the user equipment and the online service may exchange information as part of the implementation of certain methods described herein.

Embodiments in the present disclosure can be provided as methods, systems or as a combination of machine readable instructions and processing circuitry. Such machine readable instructions may be included on a non-transitory machine (for example, computer) readable storage medium (including but not limited to disc storage, CD-ROM, optical storage, etc.) having computer readable program codes therein or thereon.

The present disclosure is described with reference to flow charts and block diagrams of the method, devices and systems according to embodiments of the present disclosure. Although the flow charts described above show a specific order of execution, the order of execution may differ from that which is depicted. Blocks described in relation to one flow chart may be combined with those of another flow chart. It shall be understood that each block in the flow charts and/or block diagrams, as well as combinations of the blocks in the flow charts and/or block diagrams can be realized by machine readable instructions.

The machine readable instructions may, for example, be executed by a general purpose computer, a special purpose computer, an embedded processor or processors of other programmable data processing devices to realize the functions described in the description and diagrams. In particular, a processor or processing circuitry, or a module thereof, may execute the machine readable instructions. Thus functional modules of the apparatus 400, 500 (for example, the obtaining module 404, determining module 406 and/or user instruction module 408) and other devices described herein may be implemented by a processor executing machine readable instructions stored in a memory, or a processor operating in accordance with instructions embedded in logic circuitry. The term 'processor' is to be interpreted broadly to include a CPU, processing unit, ASIC, logic unit, or programmable gate array etc. The methods and functional modules may all be performed by a single processor or divided amongst several processors.

Such machine readable instructions may also be stored in a computer readable storage that can guide the computer or other programmable data processing devices to operate in a specific mode.

Such machine readable instructions may also be loaded onto a computer or other programmable data processing devices, so that the computer or other programmable data processing devices perform a series of operations to produce computer-implemented processing, thus the instructions executed on the computer or other programmable devices realize functions specified by block(s) in the flow charts and/or in the block diagrams.

Further, the teachings herein may be implemented in the form of a computer program product, the computer program product being stored in a storage medium and comprising a plurality of instructions for making a computer device implement the methods recited in the embodiments of the present disclosure.

Elements or steps described in relation to one embodiment may be combined with or replaced by elements or steps described in relation to another embodiment. Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A computer-implemented method (100) comprising:
obtaining (102) an indication comprising:
a skin parameter of a user, wherein the indication of the skin parameter comprises an indication of skin irritation determined by a measurement of imaging data; and
an interaction between the user's skin and a hair removal unit;
determining (104) a position of the hair removal unit relative to the user's skin;
determining (106) a hair removal instruction for the user based on the indication and the position; and
causing (108) a user interface to provide the hair removal instruction for the user.

2. The method of claim 1, wherein the skin parameter comprises a user skin condition determined, using a machine-learning model trained to identify and/or classify skin parameters, from the imaging data of the user's skin.

3. The method of claim 2, wherein the indication further comprises a hair parameter of the user, wherein the hair parameter comprises a user hair condition determined, using the machine-learning model, from the imaging data of the user's skin.

4. The method of any preceding claim, wherein the interaction between the user's skin and a hair removal unit is the pressure applied between the hair removal unit and the user's skin.

5. The method of any preceding claim, comprising causing (302) an imaging device to acquire the imaging data prior to a user hair removal session, to determine (304) pre-hair removal skin parameter data.

6. The method of claim 5, comprising:
causing (326) the imaging device to acquire the imaging data during and/or after the user hair removal session, to determine present and/or post-hair removal skin parameter data (318, 328);
generating (328) skin parameter data based on the imaging data; and
determining (330) a skin parameter map for the user based on a comparison between the pre-hair removal and present and/or post-hair removal skin parameter data.

7. The method of claim 6, wherein the skin parameter comprises a visible skin irritation indicator and is based on whether or not the comparison identifies any change in the visible skin irritation indicator between the pre-hair removal and present and/or post-hair removal skin parameter data.

8. The method of any preceding claim, wherein determining the hair removal instruction comprises:
accessing an optimal hair removal map of the user's skin, wherein a spatial location of the optimal hair removal map is associated with an optimal hair removal technique that is determined based on at least one of: pre-hair removal skin parameter data; historical data for the user; and predetermined knowledge regarding hair removal; and
determining the hair removal instruction for the spatial location based on the optimal hair removal map.

9. The method of any preceding claim, comprising:
at least one of:
determining (314), in real-time, the position of the hair removal unit relative to the user's skin;
determining (316), in real-time, the interaction between the user's skin and the hair removal unit;
determining (318), in real-time, the skin parameter; and
determining (320) a real-time hair removal instruction for the user based on at least one of: the position; the interaction; the skin parameter; historical hair removal performance data for the user; and pre-determined hair removal performance data.

10. The method of claim 9, comprising causing (322) the user interface to provide, in real-time, the hair removal instruction for the user.

11. The method of claim 9 or 10,
wherein the historical hair removal performance data for the user comprises at least one of: user skin type; user skin condition; pressure applied between the hair removal unit and the user's skin; user hair removal behavior; visible skin irritation; hair removal results; hair removal unit motion and hair removal unit operational performance, as determined from at least one previous user hair removal session, and
wherein the pre-determined hair removal performance data comprises knowledge acquired from other users and/or clinical data regarding at least one of: skin type; skin condition; pressure applied between the hair removal unit and the other user's skin; hair removal behavior; visible skin irritation; hair removal results; hair removal unit motion and hair removal unit operational performance, from which a recommended hair removal instruction for the user can be determined in order to provide improved hair removal experience as compared to a previous user hair removal session.

12. The method of any preceding claim, wherein the hair removal instruction is configured to provide a personalized recommendation for the user regarding at least one of: pressure to apply between the hair removal unit and the user's skin; hair removal unit positioning relative to the user's skin and hair removal unit motion, the method further comprising causing the user interface to provide the hair removal instruction for the user based on whether or not the user has deviated from a previously-recommended hair removal instruction.

13. The method of any preceding claim, wherein determining (314) the position of the hair removal unit relative to the user's skin comprises acquiring at least one of: imaging data of the user's skin and the hair removal unit; and motion data from a sensor on-board the hair removal unit, and wherein the position of the hair removal unit relative to the user's skin comprises at least one of: a position of a hair removal device of the hair removal unit on the user's skin; and an orientation of the hair removal device relative to the user's skin.

14. Apparatus (400) comprising processing circuitry (402), the processing circuitry comprising:
an obtaining module (404) configured to obtain an indication comprising:
a skin parameter of a user, wherein the indication of the skin parameter comprises an indication of skin irritation determined by a measurement of imaging data; and
an interaction between the user's skin and a hair removal unit;
a determining module (406) configured to:
determine a position of the hair removal unit relative to the user's skin;
a hair removal instruction for the user based on the indication and the position; and
a user instruction module (408) configured to cause a user interface to provide the hair removal instruction for the user.

15. A tangible machine-readable medium (600) storing instructions (602) which, when executed by at least one processor (604), cause the at least one processor to:
obtain (606) an indication comprising:
a skin parameter of a user, wherein the indication of the skin parameter comprises an indication of skin irritation determined by a measurement of imaging data; and
an interaction between the user's skin and a hair removal unit;
determine (608) a position of the hair removal unit relative to the user's skin;
determine (610) a hair removal instruction for the user based on the indication and the position; and
cause (612) a user interface to provide the hair removal instruction for the user.

## Patentansprüche

1. Ein durch Computer verwirklichtes Verfahren (100), umfassend:
Erhalten (102) einer Angabe, umfassend:
Einen Hautparameter eines Benutzers, wobei die Angabe des Hautparameters eine Angabe einer Hautreizung umfasst, die durch eine Messung von Bildgebungsdaten bestimmt wird; und
eine Wechselwirkung zwischen der Haut des Benutzers und einer Haarentfernungseinheit;
Bestimmen (104) einer Position der Haarentfernungseinheit relativ zu der Haut des Benutzers;
Bestimmen (106) einer Haarentfernungsanweisung für den Benutzer, basierend auf der Anzeige und der Position; und
Bewirken (108), dass eine Benutzerschnittstelle die Haarentfernungsanweisung für den Benutzer bereitstellt.

2. Verfahren nach Anspruch 1, wobei der Hautparameter eine Bestimmung des Zustands der Benutzerhaut umfasst, und dies unter Verwendung eines maschinellen Lernmodells, das darauf trainiert ist, Hautparameter zu identifizieren und/oder zu klassifizieren, und zwar aus den Bildgebungsdaten der Haut des Benutzers.

3. Verfahren nach Anspruch 2, wobei die Anzeige ferner ein Haarparameter des Benutzers umfasst, wobei der Haarparameter einen bestimmten Haarzustand des Benutzers umfasst, welcher unter Verwendung des Modells für maschinelles Lernen aus den Bildgebungsdaten der Haut des Benutzers bestimmt wird.

4. Verfahren nach einem der vorhergehenden Ansprüche, wobei die Wechselwirkung zwischen der Haut des Benutzers und einer Haarentfernungseinheit der Druck ist, der zwischen der Haarentfernungseinheit und der Haut des Benutzers ausgeübt wird.

5. Verfahren nach einem der vorhergehenden Ansprüche, umfassend: Veranlassen (302) einer BildgebungsVorrichtung zum Erfassen der Bildgebungsdaten vor einer Haarentfernungssitzung durch den Benutzer, um die Hautparameterdaten vor der Haarentfernung zu bestimmen (304) .

6. Das Verfahren nach Anspruch 5, umfassend:
Veranlassen (326) der Bildgebungsvorrichtung, die Bildgebungsdaten während und/oder nach der Haarentfernungssitzung des Benutzers zu erfassen, um die Parameterdaten (318, 328); der derzeitigen Haut und/oder der Haut nach der Haarentfernung zu bestimmen;
Erzeugen (328) von Hautparameterdaten basierend auf den Bildgebungsdaten; und
Bestimmen (330) einer Hautparameterkarte für den Benutzer, basierend auf einem Vergleich zwischen den Hautparameterdaten vor der Haarentfernung und den gegenwärtigen und/oder nach der Haarentfernung.

7. Verfahren nach Anspruch 6, wobei der Hautparameter einen Indikator sichtbarer Hautreizungen umfasst, darauf basierend, ob der Vergleich irgendeine Veränderung in dem Indikator sichtbarer Hautreizungen zwischen den Hautparameterdaten vor der Haarentfernung und den jetzigen und/oder den Hautparameterdaten nach der Haarentfernung feststellt.

8. Verfahren nach einem der vorhergehenden Ansprüche, wobei die Bestimmung der Anweisungen zur Haarentfernung umfasst:
Zugreifen auf eine optimale Haarentfernungskarte der Haut des Benutzers, wobei eine räumliche Lokalisierung der optimalen Haarentfernungskarte mit einer optimalen Haarentfernungstechnik verbunden ist, die basierend auf mindestens einem der folgenden bestimmt wird:
Hautparameterdaten vor der Haarentfernung;
Historische Daten für den Benutzer; und vorgegebenes Wissen bezüglich Haarentfernung; sowie
Bestimmen der Haarentfernungsanweisung für den räumlichen Ort, basierend auf der optimalen Haarentfernungskarte.

9. Verfahren nach einem der vorhergehenden Ansprüche, umfassend wenigstens eines von:
Bestimmen (314) der Position der Haarentfernungseinheit relativ zur Haut des Benutzers in Echtzeit;
Bestimmen (316) der Wechselwirkung zwischen der Haut des Benutzers und der Haarentfernungseinheit in Echtzeit;
Bestimmen (318) des Hautparameters in Echtzeit; und
Bestimmen (320) einer Haarentfernungsanweisung für den Benutzer, basierend auf wenigstens einem von: Position;
Wechselwirkung; Hautparameter; den historischen Haarentfernungs-Leistungsdaten für den Nutzer; und vorbestimmten Haarentfernungs-Leistungsdaten.

10. Verfahren nach Anspruch 9, umfassend das Veranlassen (322) der Benutzerschnittstelle zum Bereitstellen einer Haarentfernungsanweisung für den Benutzer in Echtzeit.

11. Das Verfahren nach Anspruch 9 oder 10,
wobei die historischen Haarentfernungsleistungsdaten für den Benutzer mindestens eines umfassen von: Hauttyp des Benutzers; Hautzustand des Benutzers; Druck, der zwischen der Haarentfernungs-Einheit und der Haut des Benutzers angewendet wird; Benutzer-Haarentfernungsverhalten; sichtbare Hautreizung; Haarentfernungs-Ergebnisse; Bewegung der Haarentfernungseinheit und Betriebsleistung der Haarentfernungseinheit, wie bestimmt aus mindestens einer vorherigen Haarentfernungssitzung des Benutzers, und wobei die vorbestimmten Haarentfernungsleistungsdaten umfassen: Von anderen Benutzern erworbenes Wissen und/oder klinische Daten in Bezug auf mindestens eines der folgenden: Hauttyp; Hauterkrankungen; zwischen dem Haarentfernungsgerät und der Haut des anderen Benutzers ausgeübter Druck; Haar-Entfernungsverhalten; sichtbare Hautreizung; Ergebnisse der Haarentfernung; Bewegung der Haarentfernungseinheit und Betriebsleistung des Haarentfernungsgeräts, aus der empfohlene Haarentfernungs-Anweisungen für den Benutzer bestimmt werden können, um eine verbesserte Haarentfernungs-Erfahrung im Vergleich zu einer vorherigen Benutzer-Haarentfernungssitzung zu schaffen.

12. Verfahren nach einem der vorhergehenden Ansprüche, wobei die Anweisung zur Haarentfernung konfiguriert ist, um eine personalisierte Empfehlung für den Benutzer in Bezug auf mindestens eines der folgenden bereitzustellen:
Druck zwischen der Haarentfernungseinheit und der Haut des Benutzers; Positionieren der Haarentfernungseinheit relativ zur Bewegung der Haut- und
Haarentfernungseinheit des Benutzers, das Verfahren umfasst ferner, zu veranlassen, dass die Benutzerschnittstelle die Haarentfernungsanweisung für den Benutzer bereitstellt, basierend darauf, ob der Benutzer von einer zuvor empfohlenen Haarentfernungsanweisung abgewichen ist oder nicht.

13. Verfahren nach einem der vorhergehenden Ansprüche, wobei die Bestimmung (314) der Position der Haarentfernungseinheit in Bezug auf die Haut des Benutzers das Erfassen von mindestens einem der folgenden umfasst: Bildgebungs-Daten der Haut des Benutzers und des Haarentfernungsgeräts; und Bewegungsdaten von einem Sensor, der in der Haarentfernungseinheit eingebaut ist, und wobei die Position der Haarentfernungseinheit relativ zur Haut des Benutzers mindestens eines umfasst von: einer Position einer Haarentfernungsvorrichtung der Haarentfernungseinheit an der Haut des Benutzers; und eine Ausrichtung des Haarentfernungsgeräts relativ zur Haut des Benutzers.

14. Apparat (400), umfassend eine Verarbeitungsschaltung (402), die Verarbeitungsschaltung umfasst:
Ein Beschaffungsmodul (404), das konfiguriert ist, um eine Angabe zu erhalten, umfassend:
Ein Hautparameter eines Benutzers, wobei die Angabe des Hautparameters eine Angabe einer Hautreizung umfasst, die durch eine Messung von Bildgebungsdaten bestimmt wird; und eine Wechselwirkung zwischen der Haut des Benutzers und einer Haarentfernungseinheit;
eine Bestimmungseinheit (406), die konfiguriert ist zum:
Bestimmen einer Position der Haarentfernungseinheit relativ zu der Haut des Benutzers;
eine Haarentfernungsanweisung für den Benutzer basierend auf der Indikation und der Position; und
ein Benutzeranweisungsmodul (408), das konfiguriert ist, um zu veranlassen, dass eine Benutzerschnittstelle dazu veranlasst wird, die Haarentfernungsanweisung für den Benutzer bereitzustellen.

15. Ein gegenständliches maschinenlesbares Medium (600), das Anweisungen (602) speichert, die, wenn von mindestens einem Prozessor (604) ausgeführt, den mindestens ein Prozessor veranlassen zum:
Erhalten (606) einer Angabe, umfassend:
Ein Hautparameter eines Benutzers, wobei die Angabe des Hautparameters eine Angabe einer Hautreizung umfasst,
die durch eine Messung von Bildgebungsdaten bestimmt wird; und eine Wechselwirkung zwischen der Haut des Benutzers und einer Haarentfernungseinheit;
Bestimmen (608) einer Position der Haarentfernungseinheit relativ zu der Haut des Benutzers;
Bestimmen (610) einer Haarentfernungsanweisung für den Benutzer, basierend auf der Indikation und der Position; und
Veranlassen (612), dass eine Benutzerschnittstelle die Haarentfernungsanweisung für den Benutzer bereitstellt.

## Revendications

1. Une méthode exécutée par un ordinateur (100) qui comprend:
l'obtention (102) d'une indication comprenant:
un paramètre de peau d'un utilisateur, où l'indication d'un paramètre de peau comprend une indication de l'irritation de la peau déterminée par une mesure d'imagerie de données; et
une interaction entre la peau d'un utilisateur et une unité d'épilation;
déterminer (104) une position d'unité d'épilation par rapport à la peau de l'utilisateur;
déterminer (106) des instructions d'épilation pour un utilisateur à partir de l'indication et la position;
et fait en sorte (108) que l'interface utilisateur fournisse des instructions d'épilation pour l'utilisateur.

2. La méthode de la revendication 1, où le paramètre de la peau comprend une affection cutanée de l'utilisateur déterminé, utilisant un modèle d'apprentissage automatique formé pour identifier et/ou les paramètres et/ou classer les paramètres de la peau, à partir des données d'imagerie de la peau de l'utilisateur.

3. La méthode de la revendication 2, où une indication comprend également un paramètre de pilosité d'un utilisateur, où le paramètre de pilosité comprend une détermination de l'état de la pilosité de l'utilisateur, en utilisant le modèle d'apprentissage automatique, à partir de l'imagerie des données de la peau de l'utilisateur.

4. La méthode selon l'une quelconque des revendications, où l'interaction entre la peau d'un utilisateur et une unité d'épilation représente la pression appliquée entre l'unité d'épilation et la peau de l'utilisateur.

5. La méthode selon l'une quelconque des revendications précédentes, comprend le fait que le (302) dispositif d'imagerie obtienne les imageries de données avant une séance d'épilation, pour déterminer (304) des données sur les paramètres de la peau avant l'épilation.

6. La méthode de la revendication 5 comprend:
le fait que (326) le dispositif d'imagerie obtienne les données d'imagerie lors et/ou après la session d'épilation, pour déterminer les présents et/ou des données de paramètres cutanés après épilation (318, 328); générer (328) des données de paramètres de peau fondés sur les données d'imagerie; et
déterminer (330) une carte des paramètres de la peau pour l'utilisateur fondés sur une comparaison entre l'épilation préalable et présente et/ou des données sur les paramètres de la peau après l'épilation.

7. La méthode de la revendication 6, où le paramètre de la peau comprend un indicateur visible d'irritation cutanée et est fondé sur le fait ou non que la comparaison identifie tout changement dans l'indicateur d'irritation visible de la peau entre avant l'épilation et après l'épilation des données de paramètres de peau de suppression.

8. La méthode selon l'une quelconque des revendications précédentes, où la détermination des instructions pour l'épilation comprend:
l'accès à une carte d'épilation optimale de la peau de l'utilisateur, où un endroit spatial de la carte d'épilation optimale est associée avec une technique optimale d'épilation qui est déterminée à partir d'au moins: des données de paramètres sur la peau avant l'épilation;
des données historiques pour l'utilisateur; et des connaissances prédéterminées en matière d'épilation; et déterminer des instructions d'épilation pour l'endroit spatial fondé sur la carte d'épilation optimale.

9. La méthode selon l'une des revendications précédentes comprend:
au moins:
la détermination (314), en temps réel, la position de l'unité d'épilation par rapport à la peau de l'utilisateur;
déterminer (316), en temps réel, l'interaction entre la peau de l'utilisateur et l'unité d'épilation;
déterminer (318), en temps réel, les paramètres de peau; et
déterminer (320) en temps réel des instructions d'épilation pour l'utilisateur fondé sur au moins; l'interaction; le paramètre de la peau; des historiques de données sur les données de performances; et des données de performance d'épilation prédéterminées.

10. La méthode selon la revendication 9, comprend le fait (322) que l'interface utilisateur fournisse, en temps réel, des instructions sur l'épilation pour l'utilisateur.

11. La méthode de la revendication 9 ou 10,
où les performances des données historiques sur les performances d'épilation pour l'utilisateur comprennent au moins: un type d'utilisateur de la peau; l'état de la peau de l'utilisateur appliquée entre l'unité d'épilation et la peau e l'utilisateur; un comportement de l'utilisateur en matière d'épilation;
des résultats d'épilation; un mouvement de l'unité d'épilation et des performances opérationnelles de l'unité d'épilation,
comme déterminée à partir d'au moins une session d'épilation
précédente, et/ou des données de performance d'épilation prédéterminées comprend des connaissances acquises auprès d'autres utilisateurs et/ou des données cliniques concernant au moins: du type de peau;
un état de la peau; la pression appliquée entre l'unité d'épilation et la peau d'un autre utilisateur; le comportement épilatoire; l'irritation visible de la peau; les résultats d'épilation;
le mouvement de l'unité d'épilation et les performances opérationnelles de l'unité d'épilation, à partir de laquelle une instruction d'épilation recommandée pour l'utilisateur peut être déterminée afin de fournir une amélioration de l'expérience d'épilation par rapport à une session d'épilation précédente.

12. La méthode selon l'une des revendications précédentes, où les instructions d'épilation sont configurées pour fournir une recommandation personnalisée pour l'utilisateur en ce qui concerne au moins: une pression à appliquer entre l'unité d'épilation et la peau d'un utilisateur; le positionnement de l'unité d'épilation par rapport à la peau de l'utilisateur et l'unité de mouvement de l'unité d'épilation, la méthode comprend également le fait que l'interface utilisateur fournisse des instructions d'épilation pour l'utilisateur fondé selon l'utilisateur a dévié à partir d'instructions d'épilation précédemment recommandées.

13. La méthode selon l'une quelconque des revendications précédentes, où la détermination (314) de la position de l'unité d'épilation par rapport à la peau de l'utilisateur comprend l'obtention d'au moins une:
donnée d'imagerie de la peau de l'utilisateur et l'unité d'épilation; et
des données de mouvement à partir d'un capteur embarqué l'unité d'épilation, et
où la position de l'unité d'épilation par rapport à la peau de l'utilisateur comprend au moins une: position d'un dispositif d'épilation de l'unité d'épilation sur la peau de l'utilisateur; et
une orientation du dispositif de l'unité épilation par rapport à la peau de l'utilisateur.

14. Un appareil (400) comprend un circuit de traitement (402), le circuit de traitement comprend:
un module d'obtention (404) configuré pour obtenir une indication comprend:
un paramètre de peau d'un utilisateur, où l'indication d'un paramètre de peau comprend une indication de l'irritation de la peau déterminée par une mesure d'imagerie de données;
et une interaction entre la peau d'un utilisateur et une unité d'épilation;
un module de détermination (406) configuré pour:
déterminer la position de l'unité d'épilation par rapport à la peau d'un utilisateur;
une instruction sur l'épilation pour l'utilisateur fondée sur l'indication et la position;
et un module d'instruction pour l'utilisateur (408) configuré pour faire en sorte que l'interface utilisateur fournisse une instruction d'épilation pour l'utilisateur.

15. Un support tangible lisible par une machine (600) stockant des instructions (602) qui, lorsqu'il est exécuté par au moins un processeur (604), fait en sorte qu'au moins un processeur:
obtienne (606) une indication comprend:
un paramètre de peau d'un utilisateur, où l'indication d'un paramètre de peau comprend une indication de l'irritation de la peau déterminée par une mesure d'imagerie de données;
et une interaction entre la peau d'un utilisateur et une unité d'épilation;
déterminer (608) une position de l'unité par rapport à la peau d'un utilisateur déterminée (610) des instructions d'épilation pour l'utilisateur fondé sur l'indication et la position; et
fait en sorte (612) qu'une interface utilisateur pour fournir des instructions d'épilation pour l'utilisateur.
